# EUROPEAN PATENT SPECIFICATION

(11) **EP 2 668 290 B1**
(45) Date of publication and mention of the grant of the patent: **31.05.2017**
(21) Application number: 12702157.4
(22) Date of filing: 27.01.2012
(51) Int. Cl.: C12Q 1/68, C12Q 1/70

(54) **RNASCOPE® HPV ASSAY FOR DETERMINING HPV STATUS IN HEAD AND NECK CANCERS AND CERVICAL LESIONS**
RNASCOPE®-HPV-ASSAY ZUR BESTIMMUNG DES HPV-STATUS IN KOPF- UND HALSTUMOREN UND ZERVIXLÄSIONEN
DOSAGE PVH RNASCOPE® POUR LA DÉTERMINATION DU STATUT PVH DANS LES CANCERS DE LA TÊTE ET DU COU ET LES LÉSIONS CERVICALES

(30) Priority: 28.01.2011 US 201161437337 P
(43) Date of publication of application: 04.12.2013
(73) Proprietor: Advanced Cell Diagnostics, Inc., Newark, CA 94560 (US)
(72) Inventor: MA, Xiao-Jun, Pleasanton, CA 94588 (US); FLANAGAN, John, Walnut Creek, CA 94598 (US); LUO, Yuling, San Ramon, CA 94582 (US)
(74) Representative: Jones Day
(86) International application number: PCT/US2012/022856
(87) International publication number: WO 2012/103414

(56) References cited:
- WO-A1-2010/060103
- WO-A1-2010/129941
- WO-A2-03/057914
- WO-A2-2007/001986
- US-A1- 2006 172 285
- US-B2- 7 709 198
- SERGE J SMEETS ET AL: "A novel algorithm for reliable detection of human papillomavirus in parafin embedded head and neck cancer specimen", INTERNATIONAL JOURNAL OF CANCER, JOHN WILEY & SONS, INC, UNITED STATES, SWITZERLAND, GERMANY, vol. 121, no. 11, 1 January 2007 (2007-01-01), pages 2465-2472, XP008148665, ISSN: 0020-7136, DOI: 10.1002/IJC.22980 cited in the application
- YANG H ET AL: "Sensitive detection of human papillomavirus in cervical, head/neck, and schistosomiasis-associated bladder malignancies", PROCEEDINGS OF THE NATIONAL ACADEMY OF SCIENCES OF USA, NATIONAL ACADEMY OF SCIENCE, WASHINGTON, DC; US, vol. 102, no. 21, 24 May 2005 (2005-05-24) , pages 7683-7688, XP002488446, ISSN: 0027-8424, DOI: 10.1073/PNAS.0406904102

## Description

### CROSS-REFERENCE TO RELATED APPLICATIONS

This application claims priority to and benefit of United States Provisional Application No. 61/437,337, filed January 28, 2011, entitled "RNAscope™ HPV for Determining HPV Status in Head and Neck Cancers".

### FIELD OF INVENTION

Provided herein are methods and kits for determining whether a head and neck cancer is related to human papillomavirus (HPV) based on the presence or the absence of E6/E7 mRNA of high-risk HPV subtypes. Also provided herein are methods and kits for diagnosing whether or not a cervical lesion in a subject is benign based on the presence or absence of E6/E7 mRNA of high-risk HPV subtypes. Further provided herein are methods for determining the progression of cervical intraepithelial neoplasm (CIN) based on the spatial pattern and expression level of E6/E7 mRNA of high-risk HPV subtypes. Also provided herein are methods for determining the risk of developing cervical cancer in a subject diagnosed with cervical lesion based on the presence or absence of E6/E7 mRNA of certain subgroups of high-risk HPV subtypes.

### BACKGROUND OF THE INVENTION

The human papillomavirus (HPV) has been shown to cause a subset of head and neck cancers (HNC), especially the squamous cell carcinoma of the oropharynx (12). HPV-associated HNC has a distinct clinical profile from that of HPV-unrelated oropharyngeal cancer. It presents in younger age and more likely male patients, who are less likely to have a history of tobacco or alcohol abuse. Compared to HPV-unrelated HNC, HPV-associated HNC is also associated with a more favorable prognosis, likely due to its higher sensitivity to current radiation and chemotherapies (3). In recent decades, the incidence of HPV-associated HNC has been increasing rapidly, probably attributable to increasing high risk sexual behaviors (4). Therefore, to better manage newly diagnosed HNC, it is currently recommended to determine HPV status in the tumor by the National Comprehensive Cancer Network (NCCN) guidelines.

Cervical cancer is one of the most common malignancies affecting women worldwide and a major cause of cancer death for women globally. Cervical cancer screening programs are effective in preventing cancer and reducing mortality (16); however, there are limitations. Cervical cancer and the pre-malignant lesions (cervical intraepithial neoplasia grades I-III (CIN I, II, and III), corresponding to mild, moderate and severe dysplasia) are caused by the human papilloma virus (17, 18). Most women who acquire HPV develop transient or subclinical infections (19, 20); very few women with HPV infections will progress to cervical intraepithial neoplasia grade II or III or cancer (19, 21-22). The major limitations of cervical cancer screening programs arise from the fact that Papanicolaou (Pap) smears and even biopsy cannot distinguish benign transient HPV infections from those HPV lesions that will progress (19, 22, 23). Current management guidelines for the treatment of CIN require that all patients follow one of the recommended protocols (24). These include cytology, colposcopy, and combinations of cytology and colposcopy and HPV DNA typing at various intervals (24, 25). The diagnosis of CIN I or HPV infection leads to multiple medical office visits and various repeat tests having to be performed to ensure that patients do not progress to higher grade lesions or cancer. There is a need to improve the diagnosis of CIN and the management of its treatment.

The current cervical cancer screening methods have several limitations. For example, the current clinically approved methods are unable to distinguish transient HPV infections from true premalignant cervical lesions. The current clinically approved methods also cannot predict the likelihood that a cervical lesion in a patient will progress to a high grade lesion or cancer. Clearly, there is a need to improve the current laboratory methods to enhance the accuracy and reliability of cervical cancer diagnoses. The invention disclosed in the present application provides answers to this need.

The best biomarker for HPV-associated tumors is the HPV E6/E7 mRNA (5), which encodes the E6 and E7 oncoproteins mainly responsible for HPV-induced oncogenesis (6). The E6 and E7 proteins inactivate the tumor suppressor proteins p53 and pRB, respectively to deregulate cell cycle control and inhibit apoptosis. Therefore, the best method for determining HPV status in the tumor is to measure the E6/E7 mRNA in tumor cells (5). However, it remains impractical in the clinical setting to quantify E6/E7 mRNA using reverse transcription real time PCR (RT-PCR) due to the cumbersome procedures of RNA extraction from formalin-fixed paraffin-embedded (FFPE) tissue and RT-PCR. Moreover, RT-PCR does not register expression in individual tumor cells, which is important for determining HPV status within the tumor. Therefore, a practical, *in situ* detection method is needed to detect E6/E7 mRNA with single cell resolution.

However, existing RNA *in situ* hybridization (ISH) methods lack sufficient sensitivity and specificity for reliable HPV E6/E7 mRNA detection in FFPE tissues. As a result, two alternative biomarkers are being used in the clinic since they can be detected *in situ* with existing methods, one measuring HPV DNA by DNA ISH and another measuring by immunohistochemistry (IHC) a surrogate protein biomarker p167, which is induced by E7-mediated pRB inactivation. However, DNA ISH has low sensitivity and p16 expression may be unspecific to HPV, leading some workers to propose a combined algorithm (8,9).

Here, we describe an RNA ISH method for HPV E6/E7 mRNA detection in HNC and cervical cancer, which uses a novel ultra-sensitive and specific RNA ISH technique called RNAscope®. RNAscope is the subject for the following issued patent and patent applications US7,709,198; US11/471,278; US60/994,415; US12/284,163; US12/660,516; US12/660,524; US61/277,563; US61/279,769; US61/283,503; US61/336,944; US61/336,947; US61/336,948; US61/310,021; US61/355,244; US61/355,246.

Although RNAscope® assay is designed to detect mRNA in situ, applying this technology platform to detect HPV subtypes in cancers such as HNC and cervical cancer is still challenging in several aspects.

First, HPV is a family of virus with more than 100 subtypes. The target sequences are highly homogonous among family members. Thus, the RNA ISH assay for detecting different subtypes of HPV must be capable of differentiating each subtype at very high specificity. For example, only a small subgroup of "high-risk" types in the family are considered oncogenic and thus need to be separated from the rest of the HPV subtypes in the family.

Second, the multiple oligo target probes required for detecting high-risk HPV subtypes make it difficult to design an RNAscope® assay. The oncogenic, high-risk HPV comprise a group of subtypes. The members of high-risk HPV subtypes in such group are different among different disease indications. For example, the head and neck cancer involves at least 7 high risk subtypes, while cervical cancer involves at least 15. Furthermore, in order to detect HPV-related cancers, it is highly desirable to "pool" the individual subtypes together into a group so that the RNA ISH assay can detect "any" of the subtypes within the group in one test. However, in RNAscope® assay, each target probe for a specific target must comprise 10-20 oligos and each target probe set requires two or more of such probes. For a target probe pool which is designed to detect 15 target nucleic acids, it requires hundreds of different oligos. The behavior of such a large number of oligos in a single *in situ* hybridization experiment has never been tested and its effect is hard to predict. Before the invention, it is unclear whether the existing RNAscope® assay can be adapted to detect multiple high homology HPV subtypes simultaneously while maintaining the high specificity characteristic of such assay.

### SUMMARY OF THE INVENTION

Therefore, a purpose of the present invention is to provide an RNA ISH method for detecting the presence of high-risk HPV subtypes in various cancers, in particular head and neck cancer and cervical cancer.

This purpose is achieved by selecting specific high-risk HPV subtypes to be used in RNAscope® HPV assays. The target probes of the RNAscope® HPV assays are designed to specifically recognize the selected high-risk HPV subtypes but not other HPV subtypes of the HPV family. The multiple target probes of the RNAscope® HPV assays are also designed not to interfere with each other.

According to one aspect, the invention concerns a method of determining whether a head and neck cancer in a human is HPV-related, comprising: (a) obtaining a sample from said human wherein said sample is in a tissue section; and (b) conducting an RNA *in situ* hybridization assay using (i) one or more target probe sets which are designed to individually hybridize to E6/E7 mRNA of one or more high-risk HPV subtypes, and (ii) a universal signal amplification system, wherein the presence of E6/E7 mRNA of said one or more high-risk HPV subtypes indicates that the head and neck cancer in said human is HPV-related.

In another aspect, the invention concerns a method for determining the progression of head and neck cancer in a human, comprising: (a) obtaining a sample from said human wherein said sample is in a tissue section; and (b) conducting an RNA ISH assay using (i) one or more target probe sets which are designed to individually hybridize to E6/E7 mRNA of one or more high-risk HPV subtypes, and (ii) a universal signal amplification system; and (c) measuring the levels of E6/E7 mRNA of said one or more high-risk HPV subtypes detected in step (b); thereby determining the progression of said head and neck cancer based on the levels of E6/E7 mRNA of said one or more high-risk HPV subtypes.

In another aspect, the invention concerns a kit for determining whether a head and neck cancer in a human is HPV-related, comprising, in a suitable container means, agents for conducting an RNA ISH assay including (i) one or more target probe sets which are designed to individually hybridize to E6/E7 mRNA of one or more high-risk HPV subtypes and (ii) a universal signal amplification system.

In another aspect, the invention concerns a method of determining whether a cervical lesion in a human is a benign lesion or a CIN lesion, comprising: (a) obtaining a sample from said human wherein said sample is in a tissue section; (b) conducting an RNA ISH assay using: (i) one or more target probe sets which are designed to individually hybridize to E6/E7 mRNA of one or more high-risk HPV subtypes, (ii) a universal signal amplification system; wherein absence of E6/E7 mRNA of all said high-risk HPV subtypes indicates a benign lesion, and the presence of E6/E7 mRNA of said one or more high-risk HPV subtypes indicates a CIN lesion.

In another aspect, the invention concerns a method for determining the progression of cervical intraepithelial neoplasm (CIN) in a human, comprising: (a) obtaining a sample from said human wherein said sample is in a tissue section; (b) conducting an RNA ISH assay using (i) one or more target probe sets which are designed to individually hybridize to E6/E7 mRNA of one or more high-risk HPV subtypes; and (ii) a universal signal amplification system; and (c) analyzing the spatial pattern and measuring the levels of the E6/E7 mRNA of said one or more high-risk HPV subtypes detected in step (b); thereby determining the progression of CIN based on the spatial pattern and levels of the E6/E7 mRNA of said one or more high-risk HPV subtypes.

In another aspect, the invention concerns a kit for determining whether a cervical lesion in a human is a benign lesion or a CIN lesion, comprising, in a suitable container means, agents for conducting an RNA ISH assay including (i) one or more target probe sets which are designed to individually hybridize to E6/E7 mRNA of one or more high-risk HPV subtypes and (ii) a universal signal amplification system.

In one embodiment, the one or more high-risk HPV subtypes mentioned above are selected from a group of 18 HPV subtypes consisting of: HPV-16, HPV-18, HPV-26, HPV-31, HPV-33, HPV-35, HPV-39, HPV-45, HPV-51, HPV-52, HPV-53, HPV-56, HPV-58, HPV-59, HPV-66, HPV-68, HPV-73, and HPV-82, or any combination thereof. In one embodiment, all of above 18 HPV subtypes are used in the RNAscope® HPV assay. In another embodiment, a set of 15 HPV subtypes of the above 18 HPV subtypes are used in the RNAscope® HPV assay, including: HPV-16, HPV-18, HPV-31, HPV-33, HPV-35, HPV-39, HPV-45, HPV-51, HPV-52, HPV-56, HPV-58, HPV-59, HPV-68, HPV-73, and HPV-82. In another embodiment, a set of 7 HPV subtypes out of the above 18 HPV subtypes are used in the RNAscope® assay assay, including: HPV-16, HPV-18, HPV-31, HPV-33, HPV-35, HPV-52, and HPV-58. In yet another embodiment, only one of the above 18 subtypes, i.e., HPV-16, is used.

In another aspect, the invention concerns a method for determining the risk of developing cervical cancer in a human diagnosed with CIN, comprising: (a) obtaining a sample from said human wherein said sample is in a tissue section; (b) conducting an RNA ISH assay using (i) one or more target probe sets which are designed to individually hybridize to E6/E7 mRNA of one or more high-risk HPV subtypes, and (ii) a universal signal amplification system; wherein said one or more high-risk HPV subtypes are organized into three groups of HPV subtypes each with a different level of risk of transforming CIN to cervical cancer; wherein the presence of E6/E7 mRNA of group (1) HPV subtypes indicates the highest risk of developing cervical cancer, the presence of E6/E7 mRNA of group (2) HPV subtypes indicates a lesser risk of developing cervical cancer, and the presence of E6/E7 mRNA of group (3) HPV subtypes indicates the least risk of developing cervical cancer.

In one embodiment, said group (1) HPV subtypes includes HPV-16; said group (2) HPV subtypes includes HPV-18, HPV-31, and HPV-33; and said group (3) HPV subtypes includes HPV-26, HPV-35, HPV-39, HPV-45, HPV-51, HPV-52, HPV-53, HPV-56, HPV-58, HPV-59, HPV-66, HPV-68, HPV-73, and HPV-82.

In a preferred embodiment, the aforementioned ISH assay is an RNAscope® assay.

### BRIEF DESCRIPTION OF THE DRAWINGS

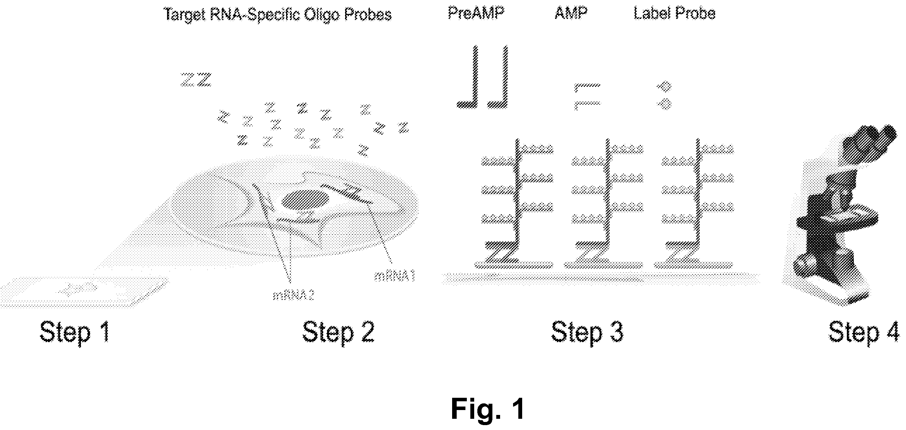

**Fig.1** illustrates the principle of the RNAscope® assay. Step 1: Cells or tissues are fixed and permeablized to allow for target probe access. Step 2: Target RNA-specific oligonucleotide target probes are hybridized to multiple mRNAs. Step 3: Multiple signal amplification molecules that each recognize a specific target probe are hybridized. Each unique label probe is conjugated to a different fluorophore or enzyme, a universal label probe is conjugated to an identical fluorophore or enzyme. Step 4: Signals are detected using a standard bright-field or epifluorescent microscope.

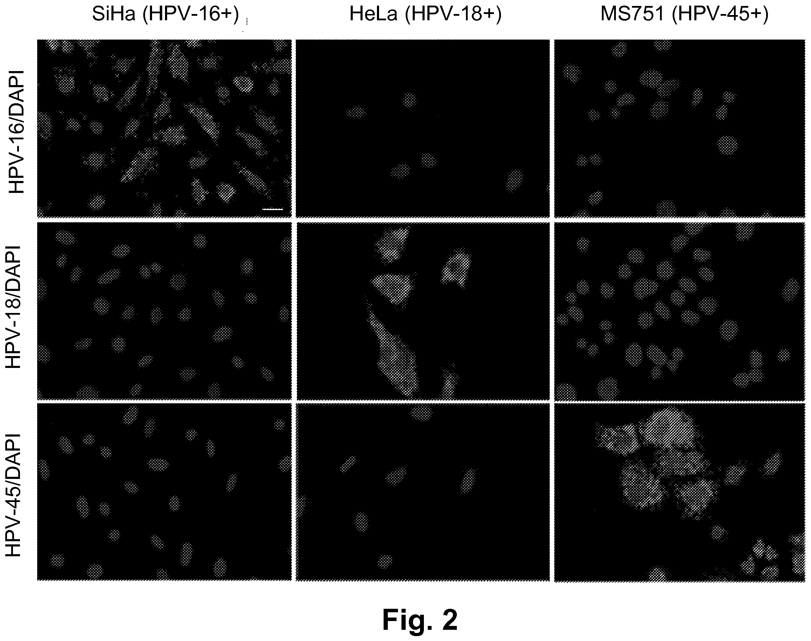

**Fig. 2** illustrates the result of feasibility test of an RNAscope® HPV assay. The RNAscope® HPV assay was designed to detect and differentiate three HPV subtypes. Target probe sets designed to hybridize specific to HPV-16, HPV-18, or HPV-45 E6/E7 mRNA are tested in various HPV-containing cell lines. The figure shows that the HPV-16 probe set produces a positive signal in SiHa cells which is known to harbor the HPV-16 subtype, the HPV-18 probe set produces a positive signal in Hela cells which is known to harbor the HPV-18 subtype, and the HPV-45 probe set produces a positive signal in MS751 cells which is known to harbor the HPV-45 subtype. Each of the above cell lines does not produce positive signals when the HPV subtypes which these cell lines do not harbor are tested.

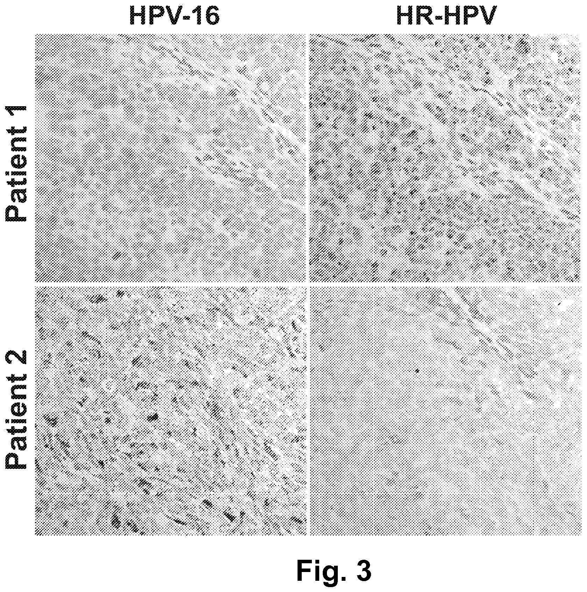

**Fig. 3** illustrates the detection of E6/E7 mRNA of high-risk HPV subtypes in FFPE tissue samples derived from HNC patients. Target probe sets which are designed to hybridize to HPV-16 (HPV-16 probe set) and target probe sets which are designed to hybridize to HPV-18, -31, -33, -35, -52, and -58 (HR-HPV probe set) are tested in HPV-infected patients. Patient 1 is a HNC patient who is known to have been infected with one or more of HPV-18, -31, -33, -35, - 52, and -58 subtypes. Patient 2 is a HNC patient who is known to have been infected with HPV-16 subtype. The figure shows that the probe sets designed in the present invention are able to detect both HNC patients. In addition, the probes sets are able to distinguish HPV-16 infected patients (lower left image) from those HNC patients who harbor other high-risk HPV subtypes (upper right image).

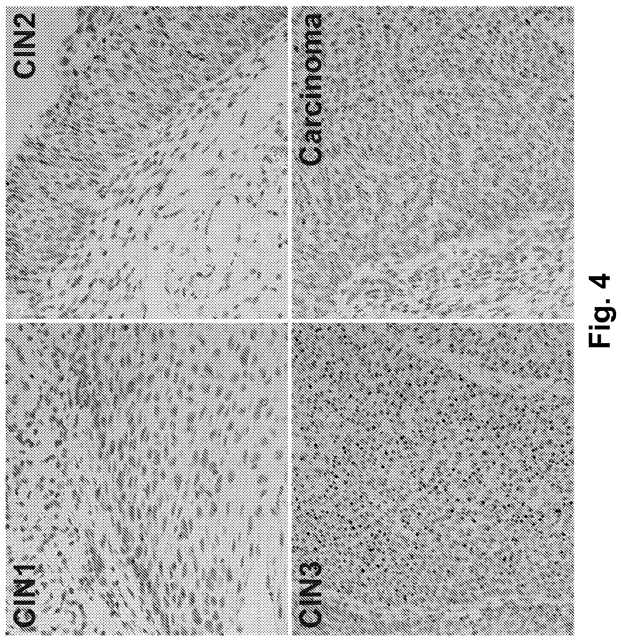

**Fig. 4** illustrates the detection of E6/E7 mRNA of high-risk HPV subtypes in FFPE tissue samples derived from patients who have been diagnosed with cervical lesions. The target probe sets pool in this experiment was designed to hybridize with HPV subtypes HPV-16, -18, -26, -31, -33, -35, -39, -45, -51, -52, -53, -56, -58, -59, -66, -68, -73, and -82. RNAscope® assay with the above target probe sets pool is conducted on cervical tissue sample of patients who have been diagnosed with cervical lesions. The figure shows the above-identified high-risk HPV subtypes were detected in patients diagnosed with CIN1, CIN2, CIN3, and malignant carcinoma. The figure also shows difference in spatial pattern and levels of E6/E7 mRNA of high-risk HPV subtypes in CIN1, CIN2, CIN3, and malignant carcinoma.

### DETAILED DESCRIPTION

The present invention relates to the use of RNAscope® assay to detect E6/E7 mRNA of one or more high-risk HPV subtypes that are known for relating to cancer. The inventors developed this mRNA *in situ* hybridization (ISH) assay based on the well known fact that some HPV subtypes are related to head and neck cancer and some HPV subtypes are related to cervical carcinoma and the precursor cervical lesions. In a HNC patient who is infected with HPV, E6/E7 mRNA of one or more of the following seven HPV subtypes are most likely to be found: HPV-16, 18, 31, 33, 35, 52, and 58 (defined here as "the HPV subgroup of 7"). Detection of any of the aforementioned seven HPV subtypes helps to determine that an HNC is HPV-related. Such determination in turn helps to determine the progression of the cancer, and to design the proper treatment. A group of eighteen HPV subtypes including HPV-16, 18, 26, 31, 33, 35, 39, 45, 51, 52, 53, 56, 58, 59, 66, 68, 73, and 82 (called here as "the HPV subgroup of 18") or a group of fifteen HPV subtypes including HPV-16, 18, 31, 33, 35, 39, 45, 51, 52, 56, 58, 59, 68, 73, and 82 (called here as "the HPV subgroup of 15") can also be used in the above test. The individual HPV subtype in the HPV subgroup of 18, or a combination of the 18 subtypes, other than the ones identified above, also have prognosis power in various degrees.

In a patient who is diagnosed with having cervical lesions, determining the present of E6/E7 mRNA of one or more of the following HPV subgroup of 18 will help to determine whether the cervical lesions are benign or related to CIN. The eighteen high-risk HPV subtypes include HPV-16, 18, 26, 31, 33, 35, 39, 45, 51, 52, 53, 56, 58, 59, 66, 68, 73, and 82. A group of fifteen HPV subtypes including HPV-16, 18, 31, 33, 35, 39, 45, 51, 52, 56, 58, 59, 68, 73 can also be used in the test of cervical lesion. The individual HPV subtype in the HPV subgroup of 18, or a combination of the 18 subtypes, other than the ones identified above, also have prognosis power on nature of cervical lesion in various degree. The presence of E6/E7 mRNA of the aforementioned high-risk HPV subtypes in a cervical lesion indicates a CIN lesion or cervical cancer (15).

### Definitions

Unless defined otherwise, all technical and scientific terms used herein have the same meaning as commonly understood by one of ordinary skill in the art to which the invention pertains. The following definitions supplement those in the art and are directed to the current application and are not to be imputed to any related or unrelated case, *e.g.,* to any commonly owned patent or application. Although any methods and materials similar or equivalent to those described herein can be used in the practice for testing of the present invention, the preferred materials and methods are described herein. Accordingly, the terminology used herein is for the purpose of describing particular embodiments only, and is not intended to be limiting.

The term "polynucleotide" (and the equivalent term "nucleic acid") encompasses any physical string of monomer units that can be corresponded to a string of nucleotides, including a polymer of nucleotides (*e.g*., a typical DNA or RNA polymer), peptide nucleic acids (PNAs), modified oligonucleotides (*e.g*., oligonucleotides comprising nucleotides that are not typical to biological RNA or DNA, such as 2'-O-methylated oligonucleotides), and the like. The nucleotides of the polynucleotide can be deoxyribonucleotides, ribonucleotides or nucleotide analogs, can be natural or non-natural, and can be unsubstituted, unmodified, substituted or modified. The nucleotides can be linked by phosphodiester bonds, or by phosphorothioate linkages, methylphosphonate linkages, boranophosphate linkages, or the like. The polynucleotide can additionally comprise non-nucleotide elements such as labels, quenchers, blocking groups, or the like. The polynucleotide can be, *e.g.*, single-stranded or double-stranded.

A "nucleic acid target" or "target nucleic acid" refers to a nucleic acid, or optionally a region thereof, that is to be detected.

A "polynucleotide sequence" or "nucleotide sequence" is a polymer of nucleotides (an oligonucleotide, a DNA, a nucleic acid, etc.) or a character string representing a nucleotide polymer, depending on context. From any specified polynucleotide sequence, either the given nucleic acid or the complementary polynucleotide sequence (*e.g*., the complementary nucleic acid) can be determined.

The term "gene" is used broadly to refer to any nucleic acid associated with a biological function. Genes typically include coding sequences and/or the regulatory sequences required for expression of such coding sequences. The term gene can apply to a specific genomic sequence, as well as to a cDNA or an mRNA encoded by that genomic sequence.

The terms "biomarker" and "marker," as used interchangeably herein, refer to both the protein/gene-product in question and the gene coding for this product.

The terms "biological sample" or "tissue sample," as used herein, refer to a sample obtained from a biological subject, including sample of biological tissue or fluid origin, obtained, reached, or collected in vivo or in situ. A biological sample also includes samples from a region of a biological subject containing precancerous or cancer cells or tissues. Such samples can be, but are not limited to, organs, tissues, fractions and cells isolated from a mammal. Exemplary biological samples include but are not limited to cell lysate, a cell culture, a cell line, a tissue, an organ, an organelle, a biological fluid, and the like. Preferred biological samples include but are not limited to a skin sample, tissue biopsies, and the like.

A "label" is a moiety that facilitates detection of a molecule. Common labels in the context of the present invention include fluorescent, luminescent, light-scattering, and/or colorimetric labels. Suitable labels include enzymes and fluorescent moieties, as well as radionuclides, substrates, cofactors, inhibitors, chemiluminescent moieties, magnetic particles, and the like. Patents teaching the use of such labels include U.S. Pat. Nos. 3,817,837; 3,850,752; 3,939,350; 3,996,345; 4,277,437; 4,275,149; and 4,366,241. Many labels are commercially available and can be used in the context of the invention.

A "target probe" is a polynucleotide that is capable of hybridizing to a target nucleic acid and capturing a label probe to that target nucleic acid. The target probe can hybridize directly to the label probe, or it can hybridize to one or more nucleic acids that in turn hybridize to the label probe; for example, the target probe can hybridize to an amplifier or a preamplifier. The target probe thus includes a first polynucleotide sequence that is complementary to a polynucleotide sequence of the target nucleic acid and a second polynucleotide sequence that is complementary to a polynucleotide sequence of the label probe, amplifier, preamplifier, or the like. The target probe is preferably single-stranded. The target probe is also called "capture probe" in U.S. Application No. 11/471,278 and "label extender" in U.S. Patent No. 7,709,198. The three terms are used interchangeably in this application.

An "amplifier" is a molecule, typically a polynucleotide, that is capable of hybridizing to multiple label probes. Typically, the amplifier hybridizes to multiple identical label probes. The amplifier also hybridizes to at least one target probe or nucleic acid bound to a target probe. For example, the amplifier can hybridize to at least one target probe and to a plurality of label probes, or to a preamplifier and a plurality of label probes. The amplifier can be, *e.g.,* a linear, forked, comb-like, or branched nucleic acid. As noted for all polynucleotides, the amplifier can include modified nucleotides and/or nonstandard internucleotide linkages as well as standard deoxyribonucleotides, ribonucleotides, and/or phosphodiester bonds. Suitable amplifiers are described, for example, in USPN 5,635,352, USPN 5,124,246, USPN 5,710,264, and USPN 5,849,481.

A "preamplifier" is a molecule, typically a polynucleotide, that serves as an intermediate between one or more target probes and amplifiers. Typically, the preamplifier hybridizes simultaneously to one or more target probes and to a plurality of amplifiers. Exemplary preamplifiers are described, for example, in USPN 5,635,352 and USPN 5,681,697.

### Applications in Head and Neck Cancer Management

One aspect of the present invention was conceptualized on the idea that detecting HPV biomarkers associated with head and neck cancer will help to determine whether or not the tested head and neck cancer is HPV-related. It is proposed that if HPV subtypes, such as HPV-16, 18, 31, 33, 35, 52, and 58, which are known to be high-risk for causing head and neck cancer are present in the sample tissue of a patient. The patient, who was known to have head and neck cancer, is likely to have the type of head and neck cancer that is HPV-related. In one embodiment of the invention, the RNAscope® HPV assay is designed for the *in situ* detection of E6/E7 mRNA of high-risk HPV subtypes in a tissue section. The tissue sample is first obtained from a patient who has been diagnosed with head and neck cancer. The tissue sample can be in a formalin fixed, paraffin embedded (FFPE) tissue section, or being captured on a solid surface by other means, or being suspended in a solution. When the sample is in a FFPE tissue section, the tissue can be treated with a tissue pretreatment agent, such as being heated in a citrate buffer followed by digestion with a protease. The purpose of pretreatment is to allow for RNA access by the target probes through the disruption of formaldehyde cross-linking created in the sample preparation procedure. A "target probe" is a polynucleotide that is capable of hybridizing to a target nucleic acid and capturing a label probe to that target nucleic acid. The target probe thus includes a first polynucleotide sequence that is complementary to a polynucleotide sequence of the target nucleic acid and a second polynucleotide sequence that is complementary to a polynucleotide sequence of signal amplification system. In a preferred embodiment, two target probes hybridize contiguously onto one target nucleic acid. The two target probes which hybridize onto a target nucleic acid is called a target probe set. After the optional pretreatment, the RNAscope® assay is performed on the tissue sample.

In the RNAscope® assay, a plurality of target probe sets are designed to hybridize to a plurality of target nucleic acids, i.e., E6/E7 mRNA of HPV subtypes. Each of the target probe sets is designed to specifically hybridize one target nucleic acid. In a preferred embodiment, the plurality of target nucleic acids include E6/E7 mRNA of HPV-16, 18, 31, 33, 35, 52, and 58. In another embodiment, only E6/E7 mRNA of HPV-16 is the target nucleic acid. HPV-16 is associated with head and neck cancer in most of the cases. In another embodiment, the rest of the HPV subtypes in the HPV subgroup of 7 are used as a group of target nucleic acids, i.e., HPV-18, 31, 33, 35, 52, and 58. In another embodiment, the plurality of target nucleic acids include E6/E7 mRNA of HPV-16, 18, 26, 31, 33, 35, 39, 45, 51, 52, 53, 56, 58, 59, 66, 68, 73, and 82 or any combination thereof. In another embodiment, the plurality of target nucleic acids include E6/E7 mRNA of HPV-16, 18, 31, 33, 35, 39, 45, 51, 52, 56, 58, 59, 68, 73, and 82. Since each target probe set recognizes one HPV subtype, the target probe sets can be used individually or in pools depending on the HPV subtypes intended to be detected in a given assay. For example, if only HPV subtype HPV-16 is intended to be detected, only the target probe set for HPV-16 is used. In another case, if only HPV subtypes HPV-16 and HPV-18 are intended to be detected, only the target probe sets for HPV-16 and HPV-18 are used.

In a preferred embodiment, each target probe set contains the same recognition sequence for a signal amplification system. Such signal amplification system is called a universal signal amplification system because it allows the HPV subtypes to be "pooled" and detected by a single signal amplification system. The benefit of using the universal signal amplification system lays in its simplicity and scalability. It allows seamless addition of new HPV subtypes without redesigning of the signal amplification system. If a new high-risk HPV subtype is identified in the future, it can be quickly added to the RNAscope® HPV assay without much change of the assay system.

The signal amplification system contains either a horseradish peroxidase (HRP) or alkaline phosphates (AP) label, which can be detected by the formation of a precipitate following incubation with DAB or Fast Red substrates, respectively. Optionally, the signal amplification system contains amplifiers and pre-amplifiers which are designed to help amplify the signals from the target nucleic acid.

In another embodiment, a distinct signal amplification system for each target nucleic acid in the pool of target nucleic acids is designed. In this case, each target probe set is designed to contain a specific recognition sequence for each different signal amplification system. This allows the differentiation of target nucleic acids based on the distinct signals emitted from the signal amplification systems which they each correspond to. Although this design loses the simplify and scalability described above, it allows differentiation of target nucleic acids when such requisite is needed.

In another embodiment, the result of the *in situ* detection of HPV E6/E7 mRNAs can be used to guide the treatment plan of head and neck cancer. For example, if the head and neck cancer is determined to be related to HPV, the treatment plan can be set up to target HPV, and more specifically, those HPV subtypes which are identified by the RNAscope® HPV assay.

In another embodiment, the present invention provides a method for determining the progression of head and neck cancer in a patient. For example, the HNC-specific RNAscope® HPV assay described above is used to quantify the levels of E6/E7 mRNAs of the high-risk HPB subtypes in the patient. Based on the levels of the E6/E7 mRNAs, the status of the HNC is then determined. If such test is performed repeatedly, the status of the HNC can be followed over a period of time and subsequently used for prognosis of the cancer and guiding its treatment.

In another embodiment, the present invention also provides a kit comprising reagents and instructions for practicing the methods described herein.

The present invention presents a novel concept of using RNAscope® assay to detect E6/E7 mRNAs of one or more high-risk HPV subtypes that are known to be related to a cancer, *e.g.,* a head and neck cancer or a cervical cancer. The assay detects a small number of HPV subtypes among more than 100 subtypes in the HPV family. The HPV subtypes are highly homologous within the family and thus make it very difficult for an ISH assay to differentiate each subtype with very high specificity. This problem is compounded by the requirement of pooling the target probes of different HPV subtypes in one assay, which requires hundreds of oligos to be included in a single reaction but still being able to bind to their own target nucleic acids but not to interference with the binding of other oligos to their targets. It is surprising that the pooled target probes designed in this invention did not interfere with each other, and the final design of the target probes is indeed capable of differentiating target HPV subtypes from non-target HPV subtypes with high specificity.

### Applications in Cervical Cancer Management

One aspect of the present invention was based on the idea that detecting HPV biomarkers associated with cervical cancer will help to determine whether a cervical lesion observed in a patient is a benign lesion or a CIN lesion. Traditionally, cervical intraepithelial neoplasm (CIN) is graded based on histology. Biopsy specimen of the cervical tissues is observed by clinicians who then grades the CIN by their morphology. When CIN grade is diagnosed, the reliability of the diagnosis is problematic, since the histologic diagnosis of CIN relies on subjective interpretation of cellular findings and morphology. Poor diagnostic accuracy and reproducibility can complicate and affect patient care. Incorrect classification of CIN grade on biopsy can lead to inappropriate follow-up or treatment of patients. Clinicians risk over-treating benign lesions or mismanaging precancerous lesions. The present invention provides a method to assist in the evaluation and diagnosis of cervical biopsy specimens based on the presence of HPV high-risk subtypes.

Currently, Pap smears obtained during cervical cancer screening suffer from the same limitations as processing of cervical biopsy on histology. Morphology of cervical cells on cytology cannot conclude on the cause of the HPV infection. Morphology changes due to benign HPV infections cannot be distinguished from malignant, precancerous HPV lesions. An advantage of the method disclosed herein is that it can test the nature of the cervical lesion on a molecular level, and thus determine whether the cervical lesion is a benign lesion or a CIN lesion. It is proposed that if any of E6/E7 mRNA of HPV subtypes of HPV-16, 18, 26, 31, 33, 35, 39, 45, 51, 52, 53, 56, 58, 59, 66, 68, 73, and 82 is detected in the cervical tissues of a patient, the cervical lesion can be ruled as a CIN lesion. If none of the aforementioned high-risk HPV subtypes is detected, the cervical lesion will be ruled as benign lesion. In other embodiments, the HPV subtypes of HPV-16, 18, 31, 33, 35, 39, 45, 51, 52, 56, 58, 59, 68, 73, and 82, either alone or in various combinations, can be used as the biomarker(s) for evaluating the cervical lesion.

In one embodiment of the invention, the RNAscope® HPV assay is designed for the *in situ* detection of E6/E7 mRNA of one or more high-risk HPV subtypes in a tissue section derived from the cervix of patients. The tissue sample is first obtained from a patient who has been diagnosed with HPV lesion. The sample can be captured in formalin fixed, paraffin embedded section, or captured on a solid surface by other means, or suspended in a solution. When a FFPE tissue section is used, the tissue is then treated with tissue pretreatment agent, such as being heated in a citrate buffer followed by digestion with a protease. After the sample is prepared, the RNAscope® assay is performed to detect the high-risk HPV subtypes in the sample.

In the RNAscope® HPV assay, a plurality of target probe sets are designed to hybridize to a plurality of target nucleic acids which are E6/E7 mRNA of high-risk HPV subtypes. In a preferred embodiment, the plurality of target nucleic acids include the HPV subgroup of 18. In another embodiment, the plurality of target nucleic acids include the HPV subgroup of 15. In another embodiment, the target nucleic acid is HPV-16 alone. Similar to the method described in the previous section regarding HNC, each target probe set is designed to recognize one HPV subtype, and the target probe sets can be used individually or in a pool, depending on whether the HPV subtypes are intended to be detected individually or indistinguishably. In a preferred embodiment, each target probe set contains an identical recognition sequence suitable for being detected by a universal signal amplification system.

The amplification system contains either a horseradish peroxidase (HRP) or alkaline phosphates (AP) label, which can be detected by the formation of a precipitate following incubation with DAB or Fast Red substrates, respectively. Optionally, the signal amplification system contains amplifiers and pre-amplifiers which are designed to help amplify the signals from the target nucleic acid. In another embodiment, the capture probe set is each designed to contain specific recognition sequence for a specific signal amplification system, and thus allowing detection of different target nucleic acids based on the different signals emitted from each specific signal amplification system.

Furthermore, the method disclosed herein can use the spatial pattern and levels of E6/E7 mRNA of one or more high-risk HPV subtypes in a patient diagnosed of cervical lesion to assess the state of progression toward full malignancy, and thereby assist in the management and follow-up treatment of such patient. Currently, there are no good methods to assist in the management of patients with low-grade cervical lesions where such lesions can either progress or regress. Currently available methods test state of progression towards full malignancy based on histological grading, which classifies intraepithelial cervical neoplasia lesions into CIN1, CIN2, CIN3 and invasive cancer (10). A classification of CIN2 or worse (CIN2+) is considered to be the threshold for aggressive surgical procedures. However, CIN2 diagnosis by histology alone is highly variable and imprecise, leading to both under- and over-treatment (11). The inability to predict which women will progress to higher-grade lesions from those women who will have regression results in all women being given the same intensive follow-up and testing, which wastes financial and medical resources and also reduces the patient's work productivity. HPV E6/E7 mRNA-based test can better define the oncogenic activity of the cervical lesion and better reflect disease progression or regression (12). Therefore, an embodiment of the present invention is to use RNAscope® HPV assay to detect the spatial pattern and levels of E6/E7 mRNA of high-risk HPV subtypes. One group of such subtypes is the HPV subgroup of 18. Another group of such subtypes is the HPV subgroup of 15. HPV-16 alone can also be used. By determining the progression of CIN in a patient, the present invention allows one to triage patients more specifically based on risk of disease progression, rather than require all patients to have multiple office visits and repeated cytology and colposcopy examinations.

In another embodiment, the present invention can be used to separately detect the presence of several groups of HPV subtypes. Each of such group contains HPV subtypes having the same risk of viral persistence and cervical cancer. It is now established that different HPV subtypes carry widely different levels of risk for causing pre-cancer (CIN3) and cancer (13, 14). The RNAscope® HPV assay can be configured to detect HPV subtypes associated with different risk levels. For example, the inventors developed a RNAscope® HPV assays which can test for three group of HPV subtypes, e.g., group 1 which contains HPV-16 alone, group 2 which contains a pool of HPV-18, 31, 33, and group 3 which contains a pool of HPV-26, 35, 39, 45, 51, 52, 53, 56, 58, 59, 66, 68, 73, and 82. The inventors have discovered that positivity of group 1 HPV subtype indicates highest level of developing cervical cancer. Positivity of group 2 HPV subtypes indicates a lesser risk of developing cervical cancer. The group 3 HPV subtypes indicates the least risk of developing cervical cancer. This test can be a highly efficient genotyping strategy that provides individualized risk assessment. By better assessing the stage of disease progression and risk for viral persistent and cancer development, the RNAscope® HPV test can thus provide objective information for optimal disease management. For example, based on the assessment of the stage of disease progression and risk for cancer development, one can select treatment options that are best for the patient. One can also optimize the frequency for repeating test by the patient.

In another embodiment, the present invention also provides a kit comprising reagents and instructions for practicing the methods described herein for detecting E6/E7 mRNA of high-risk HPV subtypes in cervical lesion.

The present invention presents a novel concept of determining whether a cervical lesion in a patient is a benign lesion or a CIN lesion based on the presence or absence of E6/E7 mRNA of high-risk HPV subtypes. Before the disclosure of the present invention, it has not been obvious to the people in the field that detecting high-risk HPV mRNA in cervical tissue specimens can have significant clinical utility in follow-on diagnosis for patients with positive screening results. The inventors of this application discovered a method for using spatial patter and level of E6/E7 mRNA of high-risk HPV subtypes in a patient to assess the state of progression toward full malignancy. The inventors of this application also established a method for using grouped E6/E7 HPV mRNA subtypes to assess the level of risk for a low-level lesion to progress to a higher level, malignant lesion and to a cancer.

### RNAscope® Assay Technology

The inventors of this application have developed an *in situ* hybridization method (United States Patent No. 7,709,198, and United States Patent Application No. 11/471,278; called RNAscope®, that allows for the direct visualization of RNA in situ. This method utilizes the oligonucleotide probe sets and novel signal amplification systems described below. The assay can be used on a variety of sample types including cultured cells, peripheral blood mononuclear cells (PBMCs), frozen tissue, and formalin-fixed paraffin embedded tissue. In addition, the assay can utilize both chromogenic and fluorescent detection reagents.

The RNAscope® assay technology provides multiplex nucleic acid assays in single cells (see **Fig. 1**). At the core of this technology is the "double Z" probe design, which allows robust amplification of specific hybridization signals without also amplifying nonspecific events. Each target probe ("Z") has a target-specific sequence, which binds to the target mRNA, a spacer, and a "tail" sequence. Two target probes (double Z) hybridize contiguously onto a target mRNA, and the two "tail" sequences form a hybridization site for the PreAmplifier. For example, the length of the hybridization site can be 28-base long. The double Z probe design ensures high fidelity of signal amplification because 1) it is highly unlikely that a pair of target probes will hybridize nonspecifically juxtaposed to each other to form a binding site for the PreAmplifier; and 2) neither tail alone can bind efficiently to the PreAmplifier under the assay conditions. The PreAmplifier, Amplifier and Label Probe are hybridized sequentially to each target probe pair, resulting in the accumulation of as many as 8,000 label molecules per 1 kb of target RNA. The Label Probe can be conjugated to either a fluorophore or a chromogenic enzyme (*e.g*., HRP), enabling viewing of hybridization signals under a standard bright-field or epifluorescent microscope, respectively. With a fluorescent Label Probe, the signals can contain at least 100-fold more fluorescent molecules than traditional RNA fluorescent ISH methods and are readily visible under a standard fluorescent microscope.

In addition, multiple signal amplifiers have been built that each recognizes a unique tail sequence on the target probes, allowing for the simultaneous visualization of multiple target RNAs. Importantly, this assay is compatible with partially degraded RNA present in archival FFPE tissues, since the double Z probe pairs target short regions of nucleotides in length.

### Using RNAscope® for Detection of HPV subtypes

The marked improvement in signal-to-noise ratio with RNAscope® allows detection of multiple RNA molecules as punctuate dots on the stained slides. To demonstrate this, an RNAscope® assay for detecting three HPV subtypes of HPV-18, HPV-31 and HPV-33 is illustrated below. It is clear that the target probe sets can be expanded or reduced to detect any reasonable number of HPV subtypes.

RNAscope® was used to detect three nucleic acid targets, HPV-18, HPV-31 and HPV-33, in a cervical tissue sample of a patient. In the methods, a sample comprising the cell is provided. The cell tested comprises, or is suspected of comprising, HPV-18, HPV-31 and HPV-33. Provided in the assay are: a first label probe comprising a first label, a second label probe comprising a second label, and a third label probe comprising a third label, wherein the signals from the three labels are distinguishable from each other. Alternatively, HPV-18, HPV-31 and HPV-33 labels are identical and the signals emitted from the three markers are indistinguishable from each other. For each of the three nucleic acid targets HPV-18, HPV-31 and HPV-33, three target probe sets, each comprising at least two target probes, are provided.

In one embodiment of the present invention, the first nucleic acid target is HPV-18, the second nucleic acid target is HPV-31 and the third nucleic acid target is HPV-33. When identical labels are used for HPV-18, HPV-31 and HPV-33, detection of label signals in a sample indicates that either HPV-18, HPV-31 or HPV-33, or all of them existed in the sample. The detection of uniformed label signals suggests the presence of HPV in the sample. When the three labels are distinguishable, detection of each individual labels provides further information regarding the expression of the individual genes in the sample.

The first target probe set is hybridized, in the cell, to the first nucleic acid target HPV-18 (when the first nucleic acid target is present in the cell), the second target probe set is hybridized, in the cell, to the second nucleic acid target HPV-31 (when the second nucleic acid target is present in the cell), and the third target probe set is hybridized, in the cell, to the third nucleic acid target HPV-33 (when the third nucleic acid target is present in the cell). In the case when the distinguishable label probes are used, the first label probe is captured to the first target probe set, the second label probe is captured to the second target probe set, and the third label probe is captured to the third target probe set, thereby capturing the first label probe to the first nucleic acid target HPV-18, the second label probe to the second nucleic acid target HPV-31 and the third label probe to the third nucleic acid target HPV-33. The first signal from the first label, the second signal from the second label and the third signal from the third label are then detected. Since the first, second and third labels are associated with their respective nucleic acid targets through the target probes, presence of the label(s) in the cell indicates the presence of the corresponding nucleic acid target(s) in the cell. The methods are optionally quantitative. Thus, an intensity of the first, second and third signal can be measured, and the intensity of the first signal can be correlated with a quantity of HPV-18, HPV-31 and HPV-33 in the cell. As another example, a signal spot can be counted for each copy of the HPV-18, HPV-31 and HPV-33 genes to quantitate them. In the case when the distinguishable label probes are used, identical label probes are captured to the three different target probes.

In one aspect, the label probes bind directly to the target probes. In another aspect, the label probes are captured to the target probes indirectly, for example, through binding of preamplifiers and/or amplifiers. Use of amplifiers and preamplifiers can be advantageous in increasing signal strength, since they can facilitate binding of large numbers of label probes to each nucleic acid target.

In the above classes of embodiments, one target probe hybridizes to each label probe, amplifier, or preamplifier. In alternative classes of related embodiments, two or more target probes hybridize to the label probe, amplifier, or preamplifier.

In embodiments in which two or more target probes are employed, the target probes preferably hybridize to nonoverlapping polynucleotide sequences in their respective nucleic acid target. The target probes can, but need not, cover a contiguous region of the nucleic acid target. Blocking probes, polynucleotides which hybridize to regions of the nucleic acid target not occupied by target probes, are optionally provided and hybridized to the target. For a given nucleic acid target, the corresponding target probes and blocking probes are preferably complementary to physically distinct, nonoverlapping sequences in the nucleic acid target, which nonoverlapping sequences are preferably, but not necessarily, contiguous. Having the target probes and optional blocking probes be contiguous with each other can in some embodiments enhance hybridization strength, remove secondary structure, and ensure more consistent and reproducible signal.

As noted, the methods are useful for multiplex detection of nucleic acids, including simultaneous detection of more than three nucleic acid targets. Thus, the cell optionally comprises or is suspected of comprising a fourth, fifth, sixth, seventh or even more nucleic acid target. For example, the method detecting a fourth nucleic acid target comprises: providing a fourth label probe comprising a fourth label, wherein a fourth signal from the fourth label can be either distinguishable or indistinguishable from the first three signals depending the degree of information to be collected, providing at least two fourth target probe, hybridizing in the cell the fourth target probe to the fourth nucleic acid target (when the fourth target is present in the cell), capturing the fourth label probe to the fourth target probe, and detecting the fourth signal from the fourth label.

In detection of nucleic acid targets in a cell, the cell is typically fixed and permeabilized before hybridization of the target probes, to retain the nucleic acid targets in the cell and to permit the target probes, label probes, etc. to enter the cell. The cell is optionally washed to remove materials not captured to one of the nucleic acid targets. The cell can be washed after any of various steps - for example, after hybridization of the target probes to the nucleic acid targets to remove unbound target probes, after hybridization of the preamplifiers, amplifiers, and/or label probes to the target probes, and/or the like.

The various capture and hybridization steps can be performed simultaneously or sequentially, in essentially any convenient order. Preferably, a given hybridization step is accomplished for all of the nucleic acid targets at the same time. For example, all the target probes (first, second, etc.) can be added to the cell at once and permitted to hybridize to their corresponding targets, the cell can be washed, amplifiers (first, second, etc.) can be hybridized to the corresponding target probes, the cell can be washed, the label probes (first, second, etc.) can be hybridized to the corresponding amplifiers, and the cell can then be washed again prior to detection of the labels. As another example, the target probes can be hybridized to the targets, the cell can be washed, amplifiers and label probes can be added together and hybridized, and the cell can then be washed prior to detection. It will be evident that double-stranded nucleic acid target(s) are preferably denatured, *e.g.*, by heat, prior to hybridization of the corresponding target probe(s) to the target(s).

In some embodiments, the cell is in suspension for all or most of the steps of the method, for ease of handling. However, the methods are also applicable to cells in solid tissue samples (*e.g.,* tissue sections) and/or cells immobilized on a substrate (*e.g.,* a slide or other surface). Thus, in one class of embodiments, the cell is in suspension in the sample comprising the cell, and/or the cell is in suspension during the hybridizing, capturing, and/or detecting steps. For example, the cell can be in suspension in the sample and during the hybridization, capture, optional washing, and detection steps. In other embodiments, the cell is in suspension in the sample comprising the cell, and the cell is fixed on a substrate during the hybridizing, capturing, and/or detecting steps. For example, the cell can be in suspension during the hybridization, capture, and optional washing steps and immobilized on a substrate during the detection step. In other embodiments, the sample comprises a tissue section.

### Example 1

### Protocol for the RNAscope® HPV assay

For each tumor sample, 5-micron tissue sections were cut and mounted on glass slides. The following steps were performed for each tumor sample:
1. Obtaining a sample from a subject. The sample can be a tissue sample obtained from a patient without preservation treatment or in a formalin fixed, paraffin embedded tissue section.
2. Optionally, if the sample is in a FFPE tissue section, the sample will be treated, first by heating the FFPE tissue section in a citrate buffer, and followed by digestion with a protease.
3. Performing an RNAscope® HPV assay on the sample, which includes the following tests:
   i) performing negative control using target probes against the bacterial gene dapB;
   ii) performing positive control using target probes against human gene UBC;
   iii) performing a test using HPV target probe sets containing either a pool of high-risk HPV subtypes or individual target probe set specific to one of these subtypes. For example, for head and neck cancer, a single pool of 7 high-risk types (HPV-16, 18, -31, -33, -35, -52, and -58) can be used together. Alternatively, one can split the HPV probe sets into two separate assays: one for HPV-16 alone, and another containing all other 6 types (HPV-18, -31,-33, -35, -52, and -58). The exact pooling and splitting strategy can be adapted to the specific diagnostic information desired.

This experimental protocol allows for the assessment of HPV positivity in the context of RNA integrity (UbC) and assay background (DapB). A sample is considered HPV positive if the signals emitted from the HPV sample are higher than the DapB sample. A sample is considered HPV negative if no signal is observed in the HPV sample but the signals in UbC sample is positive.

### Example 2

### RNAscope® Detection of E6/E7 mRNA of HPV Subtypes in Subtype-specific Cell Lines

In order to demonstrate the feasibility of the RNAscope® HPV assay to detect and discriminate among different HPV subtypes, an experiment was conducted to detect HPV subtypes in cell lines which are known to contain only specific HPV subtypes. The protocol for the RNAscope® HPV assay described in Example 1 was used. The target probe sets are designed to be specific for E6/E7 mRNA of HPV-16, HPV-18, and HPV-45, respectively. Hybridization of the probe sets to their target was detected using an alkaline phosphatase conjugated signal amplification system followed by development with Fast Red, which results in a red, fluorescent precipitate. As shown in **Figure 2**, the HPV-16 target probe set produced a positive signal only in SiHa and CaSki cells, both of which harbor only the HPV-16 subtype. The HPV-18 target probe set produced a positive signal only in Hela cell lines which harbor only the HPV-18 subtype. HPV-45 target probe sets only produced positive signals in the MS-751 cell lines which harbor only the HPV-45 subtype. Taken together, these results demonstrate that the probe set designed in the present invention accomplished its intended result by detecting specific HPV subtypes. In addition, these results show that the probe sets are capable of distinguishing highly homologous sequences because the tested HPV subtypes are up to 85% identical.

### Example 3

### RNAscope® Detection of HPV Subtypes in Head and Neck Cancer Tissue Samples

The following experiment was conducted to detect E6/E7 mRNA of HPV high-risk subtypes in FFPE tissue samples obtained from HNC patients. In the experiment, the protocol for the RNAscope® HPV assay described in Example 1 was used. The two groups of target probe sets were created. In one group, the target probe sets contain only target probes that only bind to HPV-16. In the other group, the target probe sets contain target probes that bind to HPV-18, 31, 33, 35, 52, and 58 (called "HR-HPV probe set"). Multiple HNC patient samples who are diagnosed with cervical lesions were tested using the above groups of target probe sets. An exemplary result is shown in **Figure 3****.** The result shows detection of positive E6/E7 mRNA of HPV high-risk subgroup in both patients. Both patients are known to have HPV-related HNC. Thus, the test proves that the RNAscope® HPV assay with the specifically designed HPV target probe sets can accurately detect those HPV subtypes and thus determine whether or not a given HNC is HPV-related. In addition, the two groups of target probe sets were used to distinguish patients harboring HPV-16 (lower left corner of **Figure 3**) from those harboring other subtypes of HPV (upper right corner of **Figure 3**). This allows further separation of different HPV genotypes. It has been known that different genotypes carry widely different levels of risk for cancer progression. The genotyping strategy disclosed in this example provides individualized risk assessment of head and neck patients.

### Example 4

### RNAscope® Detection of HPV Subtypes in Cervical Lesion Tissue Samples

The following experiment was conducted to detect E6/E7 mRNA of certain high-risk HPV subtypes in cervical tissue samples obtained from patients who have been diagnosed to have cervical lesions. A tissue microarray containing multiple cases of normal cervix tissue, CIN1, CIN2, CIN3, and malignant carcinoma was stained with a probe set pool designed to recognize the HPV subgroup of 18. In the experiment, the protocol for the RNAscope® HPV assay described in Example 1 was used. As shown in **Figure 4****,** E6/E7 mRNAs of HPV subgroup of 18 were detected in CIN1, CIN2, CIN3, and malignant carcinoma, but not in normal cervix tissues. The experiment shows that positivity of HPV E6/E7 mRNA correlates with the malignancy of cervical lesion which is diagnosed by histology method. It was also observed in **Figure 4** that the spatial pattern and expression level of HPV E6/E7 mRNA (quantified by the number of precipitation dots in the image) vary among CIN1, CIN2, CIN3, and malignant carcinoma. As disclosed in the application herein, the morphology of E6/E7 RNAscope® HPV assay image helps to define the oncogenic activity of the HPV infection in the lesion and thus allows better assessment of disease progression.

### References

1. Chung, C.H. & Gillison, M.L. Human papillomavirus in head and neck cancer: its role in pathogenesis and clinical implications. Clinical cancer research : an official journal of the American Association for Cancer Research 15, 6758-62(2009).
2. D'Souza, G. et al. Case-control study of human papillomavirus and oropharyngeal cancer. The New England journal of medicine 356, 1944-56(2007).
3. Ang, K.K. et al. Human papillomavirus and survival of patients with oropharyngeal cancer. The New England journal of medicine 363, 24-35(2010).
4. Marur, S. et al. HPV-associated head and neck cancer: a virus-related cancer epidemic. The Lancet Oncology 11, 781-789(2010).
5. Shi, W. et al. Comparative prognostic value of HPV16 E6 mRNA compared with in situ hybridization for human oropharyngeal squamous carcinoma. Journal of clinical oncology : official journal of the American Society of Clinical Oncology 27, 6213-21(2009).
6. Moody, C.A, Laimins, L.A. Human papillomavirus oncoproteins: pathways to transformation. Nature reviews. Cancer (2010).doi:10.1038/nrc2886
7. Lewis, J.S. et al. p16 positive oropharyngeal squamous cell carcinoma: an entity with a favorable prognosis regardless of tumor HPV status. The American journal of surgical pathology 34, 1088-96(2010).
8. Robinson, M., Sloan, P. & Shaw, R. Refining the diagnosis of oropharyngeal squamous cell carcinoma using human papillomavirus testing. Oral oncology 46, 492-6(2010).
9. Smeets, S.J. et al. A novel algorithm for reliable detection of human papillomavirus in paraffin embedded head and neck cancer specimen. International journal of cancer. Journal international du cancer 121, 2465-72(2007).
10. Schiffman, M. et al. Human papillomavirus and cervical cancer. Lancet 370, 890-907(2007).
11. Stoler, M.H. & Schiffman, M. Interobserver reproducibility of cervical cytologic and histologic interpretations: realistic estimates from the ASCUS-LSIL Triage Study. JAMA: the journal of the American Medical Association 285, 1500-5(2001).
12. Ho, C.-M. et al. Type-specific human papillomavirus oncogene messenger RNA levels correlate with the severity of cervical neoplasia. International journal of cancer. Journal international du cancer 127, 622-32(2010).
13. Kjær, S.K. et al. Long-term Absolute Risk of Cervical Intraepithelial Neoplasia Grade 3 or Worse Following Human Papillomavirus Infection: Role of Persistence. Journal of the National Cancer Institute 102, 1478-88(2010).
14. Meijer, C.J., Snijders, P.J. & Castle, P.E. Clinical utility of HPV genotyping. Gynecologic oncology 103, 12-7(2006).
15. Naucler, P. et al. Human papillomavirus and Papanicolaou tests to screen for cervical cancer. The New England journal of medicine 357, 1589-97(2007).
16. Landis et al., Cancer statistics, 1999. CA Cancer J Clin 1999; 49(1):8-31.
17. Wright et al., Precancerous lesions of the cervix. In: Kurman R J, ed. Blausteins pathology of the female genital tract. 5th ed. New York: Springer; 2002:253-324.
18. Well, Human papillomavirus associated lesions of the lower female genital tract. In: Lowe D, Fox H, Editors. Advances in Gynacological Pathology. UK: Churchill Livingstone, 1992: 79-97.
>Sp Syrjanen, Spontaneous evolution of intraepithelial lesions according to the grade and type of the implicated human papillomavirus (HPV). Eur J Obstet Gynecol Reprod Biol 1996; 65(1):45-53.
20. Hines et al., Human Papillomaviruses: Their clinical significance in the management of cervical carcinoma. Oncology 1995; 9:279-85.
21. Solomon et al., Cervical cancer screening rates in the United States and the potential impact of implementation of screening guidelines. CA Cancer J Clin 2007; 57(2):105-11.
22. Ostor, Natural history of cervical intraepithelial neoplasia: a critical review. Int J Gynecol Pathol 1993; 12(2): 186-92.
23. Thomison et al., Human papillomavirus: molecular and cytologic/histologic aspects related to cervical intraepithelial neoplasia and carcinoma. Hum Pathol. 2008; 39(2): 154-66.
24. Wright et al., 2006 American Society for Colposcopy and Cervical Pathology-sponsored Consensus Conference. 2006 consensus guidelines for the management of women with cervical intraepithelial neoplasia or adenocarcinoma in situ. Am J Obstet Gynecol 2007; 197(4):340-5.
25. Wright et al., 2006 American Society for Colposcopy and Cervical Pathology-sponsored Consensus Conference. 2006 consensus guidelines for the management of women with cervical intraepithelial neoplasia or adenocarcinoma in situ. J Low Genit Tract Dis 2007; 11(4):223-39.

## Claims

1. A method of determining whether a head and neck cancer in a human is HPV-related, comprising conducting an RNA *in situ* hybridization (ISH) assay on a head and neck cancer sample obtained from said human, wherein said sample is in a tissue section, wherein said ISH assay comprises:
(i) more than one target probe sets which are designed to individually hybridize to E6/E7 mRNA of more than one high-risk HPV subtypes, and
(ii) a universal signal amplification system,
wherein each target probe set contains a plurality of target probes,
wherein each target probe within one target probe set comprises a first polynucleotide sequence that is complementary to E6/E7 mRNA of one high-risk HPV subtype, and a second polynucleotide sequence that is complementary to a polynucleotide sequence of the universal signal amplification system, and
wherein said second polynucleotide sequence is identical across different target probe sets, and
wherein the presence of E6/E7 mRNA of said one or more high-risk HPV subtypes indicates that the head and neck cancer in said human is HPV-related.

2. A method for determining the progression of head and neck cancer in a human, comprising:
(a) conducting an RNA *in situ* hybridization (ISH) assay on a head and neck cancer sample obtained from said human, wherein said sample is in a tissue section, wherein said ISH assay comprises:
(i) more than one target probe sets which are designed to individually hybridize to E6/E7 mRNA of more than one high-risk HPV subtypes, and
(ii) a universal signal amplification system; and
(b) measuring the levels of E6/E7 mRNA of said one or more high-risk HPV subtypes detected in step (a);
wherein each target probe set contains a plurality of target probes,
wherein each target probe within one target probe set comprises a first polynucleotide sequence that is complementary to E6/E7 mRNA of one high-risk HPV subtype, and a second polynucleotide sequence that is complementary to a polynucleotide sequence of the universal signal amplification system, and
wherein said second polynucleotide sequence is identical across different target probe sets, and
wherein the levels of E6/E7 mRNA of said one or more high-risk HPV subtypes determines the progression of said head and neck cancer.

3. A method of determining whether a cervical lesion in a human is a benign lesion or a cervical intraepithelial neoplasm (CIN) lesion, comprising conducting an RNA *in situ* hybridization (ISH) assay on a cervical tissue sample obtained from said human, wherein said sample is in a tissue section, wherein said ISH assay comprises:
(i) more than one target probe sets which are designed to individually hybridize to E6/E7 mRNA of more than one high-risk HPV subtypes,
(ii) a universal signal amplification system;
wherein each target probe set contains a plurality of target probes,
wherein each target probe within one target probe set comprises a first polynucleotide sequence that is complementary to E6/E7 mRNA of one high-risk HPV subtype, and a second polynucleotide sequence that is complementary to a polynucleotide sequence of the universal signal amplification system, and
wherein said second polynucleotide sequence is identical across different target probe sets, and
wherein absence of E6/E7 mRNA of all said high-risk HPV subtypes indicates a benign lesion, and the presence of E6/E7 mRNA of said one or more high-risk HPV subtypes indicates a CIN lesion.

4. A method for determining the progression of cervical intraepithelial neoplasm (CIN) in a human, comprising:
(a) conducting an RNA *in situ* hybridization (ISH) assay on a cervical tissue sample obtained from said human, wherein said sample is in a tissue section, wherein said ISH assay comprises:
(i) more than one target probe sets which are designed to individually hybridize to E6/E7 mRNA of more than one high-risk HPV subtypes, and
(ii) a universal signal amplification system; and
(b) analyzing the spatial pattern and measuring the levels of the E6/E7 mRNA of said one or more high-risk HPV subtypes detected in step (a);
wherein each target probe set contains a plurality of target probes,
wherein each target probe within one target probe set comprises a first polynucleotide sequence that is complementary to E6/E7 mRNA of one high-risk HPV subtype, and a second polynucleotide sequence that is complementary to a polynucleotide sequence of the universal signal amplification system, and
wherein said second polynucleotide sequence is identical across different target probe sets, and
wherein the spatial pattern and levels of the E6/E7 mRNA of said one or more high-risk HPV subtypes determines the progression of CIN.

5. A method for determining the risk of developing cervical cancer in a human diagnosed with cervical intraepithelial neoplasm (CIN), comprising conducting an RNA *in situ* hybridization (ISH) assay on a cervical tissue sample obtained from said human, wherein said sample is in a tissue section, wherein said ISH assay comprises:
(i) more than one target probe sets which are designed to individually hybridize to E6/E7 mRNA of more than one high-risk HPV subtypes, and
(ii) a universal signal amplification system;
wherein each target probe set contains a plurality of target probes,
wherein each target probe within one target probe set comprises a first polynucleotide sequence that is complementary to E6/E7 mRNA of one high-risk HPV subtype, and a second polynucleotide sequence that is complementary to a polynucleotide sequence of the universal signal amplification system, and
wherein said second polynucleotide sequence is identical across different target probe sets,
wherein said one or more high-risk HPV subtypes are organized into three groups of HPV subtypes each with a different level of risk of transforming CIN to cervical cancer, and
wherein the presence of E6/E7 mRNA of group (1) HPV subtypes indicates the highest risk of developing cervical cancer, the presence of E6/E7 mRNA of group (2) HPV subtypes indicates a lesser risk of developing cervical cancer, and the presence of E6/E7 mRNA of group (3) HPV subtypes indicates the least risk of developing cervical cancer; and wherein said group (1) HPV subtypes includes HPV-16; said group (2) HPV subtypes includes HPV-18, HPV-31, and HPV-33; and said group (3) HPV subtypes includes HPV-26, HPV-35, HPV-39, HPV-45, HPV-51, HPV-52, HPV-53, HPV-56, HPV-58, HPV-59, HPV-66, HPV-68, HPV-73, and HPV-82.

6. The method of any one of claims 1 to 5, wherein said tissue section is a formalin fixed, paraffin embedded tissue section.

7. A kit comprising, in a suitable container means, agents for conducting an RNA *situ* hybridization (ISH) assay including
(i) more than one target probe sets which are designed to individually hybridize to E6/E7 mRNA of more than one high-risk HPV subtypes, and
(ii) a universal signal amplification system,
wherein each target probe set contains a plurality of target probes,
wherein each target probe within one target probe set comprises a first polynucleotide sequence that is complementary to E6/E7 mRNA of one high-risk HPV subtype, and a second polynucleotide sequence that is complementary to a polynucleotide sequence of the universal signal amplification system, and
wherein said second polynucleotide sequence is identical across different target probe sets, and
wherein the kit is for determining whether a head and neck cancer in a human is HPV-related, or whether a cervical lesion in a human is a benign lesion or a cervical intraepithelial neoplasm (CIN) lesion.

8. The method of any one of claims 1 to 6, or the kit of claim 7, wherein said universal signal amplification system contains either a horseradish peroxidase (HRP) or alkaline phosphatase (AP) label, which can be detected by the formation of a precipitate following incubation with DAB or Fast Red substrates, respectively.

9. The method of any one of claims 1 to 6, or the kit of claim 7, wherein said universal signal amplification system contains amplifiers and pre-amplifiers which are designed to help amplify the signals from the E6/E7 mRNA.

10. The method of any one of claims 1 to 9, or the kit of any one of claims 7 to 9, wherein said one or more high-risk HPV subtypes are selected from a group consisting of HPV-16, HPV-18, HPV-26, HPV-31, HPV-33, HPV-35, HPV-39, HPV-45, HPV-51, HPV-52, HPV-53, HPV-56, HPV-58, HPV-59, HPV-66, HPV-68, HPV-73, and HPV-82, or any combination thereof.

11. The method or kit of claim 10, wherein said group consists of HPV subtypes of: HPV-16, HPV-18, HPV-26, HPV-31, HPV-33, HPV-35, HPV-39, HPV-45, HPV-51, HPV-52, HPV-53, HPV-56, HPV-58, HPV-59, HPV-66, HPV-68, HPV-73, and HPV-82.

12. The method or kit of claim 10, wherein said group consists of HPV subtypes of: HPV-16, HPV-18, HPV-31, HPV-33, HPV-35, HPV-39, HPV-45, HPV-51, HPV-52, HPV-56, HPV-58, HPV-59, HPV-68, HPV-73, and HPV-82.

13. The method or kit of claim 10, wherein said group consists of HPV subtypes of: HPV-16, HPV-18, HPV-31, HPV-33, HPV-35, HPV-52, and HPV-58.

14. The method or kit of claim 10, wherein said group consists of HPV subtype HPV-16.

## Patentansprüche

1. Verfahren zum Bestimmen, ob ein Kopf- und Hals-Krebs bei einem Menschen HPV-bedingt ist, umfassend Durchführen eines RNA-*in situ-*Hybridisierungs (ISH)-Assays an einer von dem Menschen erhaltenen Kopf- und Hals-Krebs-Probe, wobei die Probe in einem Gewebe-Schnitt ist, wobei das ISH-Assay umfasst:
(i) mehr als einen an Target-Sonden-Sätzen, welche zum individuellen Hybridisieren mit E6/E7 mRNA von mehr als einem Hoch-Risiko-HPV-Subtypen ausgelegt sind, und
(ii) ein Universal-Signal-Amplifizierungs-System,
wobei jeder Target-Sonden-Satz eine Vielzahl von Target-Sonden enthält, wobei jede Target-Sonde in einem Target-Sonden-Satz eine erste Polynukleotid-Sequenz, die zu E6/E7 mRNA von einem Hoch-Risiko-HPV-Subtyp komplementär ist, und eine zweite Polynukleotid-Sequenz, die zu einer Polynukleotid-Sequenz von dem Universal-Signal-Amplifizierungs-System komplementär ist, umfasst und
wobei die zweite Polynukleotid-Sequenz über verschiedene Target-Sonden-Sätze identisch ist, und
wobei das Vorliegen von E6/E7 mRNA von dem einen oder mehreren Hoch-Risiko-HPV-Subtypen anzeigt, dass der Kopf- und Hals-Krebs in dem Menschen HPV-bedingt ist.

2. Verfahren zum Bestimmen des Fortschritts von Kopf- und Hals-Krebs bei einem Menschen, umfassend:
(a) Durchführen eines RNA-*in situ*-Hybridisierungs (ISH)-Assays an einer von dem Menschen erhaltenen Kopf- und Hals-Krebs-Probe, wobei die Probe in einem Gewebe-Schnitt ist, wobei das ISH-Assay umfasst:
(i) mehr als einen an Target-Sonden-Sätzen, welche zum individuellen Hybridisieren mit E6/E7 mRNA von mehr als einem Hoch-Risiko-HPV-Subtypen ausgelegt sind, und
(ii) ein Universal-Signal-Amplifizierungs-System; und
(b) Messen der Mengen von E6/E7 mRNA von dem einen oder mehreren in Schritt (a) nachgewiesenen Hoch-Risiko-HPV-Subtypen;
wobei jeder Target-Sonden-Satz eine Vielzahl von Target-Sonden enthält,
wobei jede Target-Sonde in einem Target-Sonden-Satz eine erste Polynukleotid-Sequenz, die zu E6/E7 mRNA von einem Hoch-Risiko-HPV-Subtyp komplementär ist, und eine zweite Polynukleotid-Sequenz, die zu einer Polynukleotid-Sequenz von dem Universal-Signal-Amplifizierungs-System komplementär ist, umfasst und
wobei die zweite Polynukleotid-Sequenz über verschiedene Target-Sonden-Sätze identisch ist, und
wobei die Mengen von E6/E7 mRNA von dem einen oder mehreren Hoch-Risiko-HPV-Subtypen den Fortschritt von dem Kopf- und Hals-Krebs bestimmt.

3. Verfahren zum Bestimmen, ob eine Zervixläsion bei einem Menschen eine benigne Läsion oder eine zervikale intraepitheliale Neoplasie (CIN)-Läsion ist, umfassend Durchführen eines RNA-*in situ*-Hybridisierungs (ISH)-Assays an einer von dem Menschen erhaltenen zervikalen Gewebe-Probe, wobei die Probe in einem Gewebe-Schnitt ist, wobei das ISH-Assay umfasst:
(i) mehr als einen an Target-Sonden-Sätzen, welche zum individuellen Hybridisieren mit E6/E7 mRNA von mehr als einem Hoch-Risiko-HPV-Subtypen ausgelegt sind,
(ii) ein Universal-Signal-Amplifizierungs-System;
wobei jeder Target-Sonden-Satz eine Vielzahl von Target-Sonden enthält,
wobei jede Target-Sonde in einem Target-Sonden-Satz eine erste Polynukleotid-Sequenz, die zu E6/E7 mRNA von einem Hoch-Risiko-HPV-Subtyp komplementär ist, und eine zweite Polynukleotid-Sequenz, die zu einer Polynukleotid-Sequenz von dem Universal-Signal-Amplifizierungs-System komplementär ist, umfasst und
wobei die zweite Polynukleotid-Sequenz über verschiedene Target-Sonden-Sätze identisch ist, und
wobei die Abwesenheit von E6/E7 mRNA von allen den Hoch-Risiko-HPV-Subtypen eine benigne Läsion anzeigt, und das Vorliegen von E6/E7 mRNA von dem einen oder mehreren Hoch-Risiko-HPV-Subtypen eine CIN-Läsion anzeigt.

4. Verfahren zum Bestimmen des Fortschritts von einer zervikaler intraepithelialer Neoplasie (CIN) bei einem Menschen, umfassend:
(a) Durchführen eines RNA-*in situ*-Hybridisierungs (ISH)-Assays an einer von dem Menschen erhaltenen zervikalen Gewebe-Probe, wobei die Probe in einem Gewebe-Schnitt ist, wobei das ISH-Assay umfasst:
(i) mehr als einen an Target-Sonden-Sätzen, welche zum individuellen Hybridisieren mit E6/E7 mRNA von mehr als einem Hoch-Risiko-HPV-Subtypen ausgelegt sind, und
(ii) ein Universal-Signal-Amplifizierungs-System; und
(b) Analysieren der Raumstruktur und Messen der Mengen der E6/E7 mRNA von dem einen oder mehreren in Schritt (a) nachgewiesenen Hoch-Risiko-HPV-Subtypen;
wobei jeder Target-Sonden-Satz eine Vielzahl von Target-Sonden enthält,
wobei jede Target-Sonde in einem Target-Sonden-Satz eine erste Polynukleotid-Sequenz, die zu E6/E7 mRNA von einem Hoch-Risiko-HPV-Subtyp komplementär ist, und eine zweite Polynukleotid-Sequenz, die zu einer Polynukleotid-Sequenz von dem Universal-Signal-Amplifizierungs-System komplementär ist, umfasst und
wobei die zweite Polynukleotid-Sequenz über verschiedene Target-Sonden-Sätze identisch ist, und
wobei die Raumstruktur und Mengen von der E6/E7 mRNA von dem einen oder mehreren Hoch-Risiko-HPV-Subtypen den Fortschritt von einer CIN bestimmt.

5. Verfahren zum Bestimmen des Risikos der Entwicklung von Zervix-Karzinom bei einem Menschen, bei dem eine zervikale intraepitheliale Neoplasie (CIN) diagnostiziert wurde, umfassend Durchführen eines RNA-*in situ-*Hybridisierungs (ISH)-Assays an einer von dem Menschen erhaltenen zervikalen Gewebe-Probe, wobei die Probe in einem Gewebe-Schnitt ist, wobei das ISH-Assay umfasst:
(i) mehr als einen an Target-Sonden-Sätzen, welche zum individuellen Hybridisieren mit E6/E7 mRNA von mehr als einem Hoch-Risiko-HPV-Subtypen ausgelegt sind, und
(ii) ein Universal-Signal-Amplifizierungs-System;
wobei jeder Target-Sonden-Satz eine Vielzahl von Target-Sonden enthält,
wobei jede Target-Sonde in einem Target-Sonden-Satz eine erste Polynukleotid-Sequenz, die zu E6/E7 mRNA von einem Hoch-Risiko-HPV-Subtyp komplementär ist, und eine zweite Polynukleotid-Sequenz, die zu einer Polynukleotid-Sequenz von dem Universal-Signal-Amplifizierungs-System komplementär ist, umfasst und
wobei die zweite Polynukleotid-Sequenz über verschiedene Target-Sonden-Sätze identisch ist,
wobei die einen oder mehreren Hoch-Risiko-HPV-Subtypen in drei Gruppen von HPV-Subtypen jeder mit einem anderen Risikograd der Umwandlung von CIN zu Zervix-Karzinom geordnet werden, und
wobei das Vorliegen von E6/E7 mRNA von Gruppe (1) HPV-Subtypen das höchste Risiko der Entwicklung von Zervix-Karzinom anzeigt, das Vorliegen von E6/E7 mRNA von Gruppe (2) HPV-Subtypen ein geringeres Risiko der Entwicklung von Zervix-Karzinom anzeigt, und das Vorliegen von E6/E7 mRNA von Gruppe (3) HPV-Subtypen das geringste Risiko der Entwicklung von Zervix-Karzinom anzeigt; und wobei die Gruppe (1) HPV-Subtypen HPV-16 einschließt; die Gruppe (2) HPV-Subtypen HPV-18, HPV-31 und HPV-33 einschließt; und die Gruppe (3) HPV-Subtypen HPV-26, HPV-35, HPV-39, HPV-45, HPV-51, HPV-52, HPV-53, HPV-56, HPV-58, HPV-59, HPV-66, HPV-68, HPV-73 und HPV-82 einschließt.

6. Verfahren nach einem der Ansprüche 1 bis 5, wobei der Gewebe-Schnitt ein in Formalin fixierter, Paraffin-eingebetteter Gewebe-Schnitt ist.

7. Kit, umfassend in einer geeigneten Behältereinrichtung, Mittel zum Durchführen eines RNA-*in situ*-Hybridisierungs (ISH)-Assays, das einschließt
(i) mehr als einen an Target-Sonden-Sätzen, welche zum individuellen Hybridisieren mit E6/E7 mRNA von mehr als einem Hoch-Risiko-HPV-Subtypen ausgelegt sind, und
(ii) ein Universal-Signal-Amplifizierungs-System,
wobei jeder Target-Sonden-Satz eine Vielzahl von Target-Sonden enthält,
wobei jede Target-Sonde in einem Target-Sonden-Satz eine erste Polynukleotid-Sequenz, die zu E6/E7 mRNA von einem Hoch-Risiko-HPV-Subtyp komplementär ist, und eine zweite Polynukleotid-Sequenz, die zu einer Polynukleotid-Sequenz von dem Universal-Signal-Amplifizierungs-System komplementär ist, umfasst und
wobei die zweite Polynukleotid-Sequenz über verschiedene Target-Sonden-Sätze identisch ist, und
wobei das Kit zum Bestimmen ist, ob ein Kopf- und Hals-Krebs bei einem Menschen HPV-bedingt ist, oder ob eine Zervixläsion bei einem Menschen eine benigne Läsion oder eine zervikale intraepitheliale Neoplasie (CIN)-Läsion ist.

8. Verfahren nach einem der Ansprüche 1 bis 6, oder Kit nach Anspruch 7, wobei das Universal-Signal-Amplifizierungs-System entweder eine Meerettichperoxidase (HRP)- oder alkalische Phosphatase (AP)-Markierung enthält, welche durch die Bildung eines Niederschlags nach Inkubation mit DAB bzw. Fast-Red-Substraten nachgewiesen werden kann.

9. Verfahren nach einem der Ansprüche 1 bis 6, oder Kit nach Anspruch 7, wobei das Universal-Signal-Amplifizierungs-System Amplifier und Preamplifier enthält, welche zum Unterstützen der Amplifizierung der Signale der E6/E7 mRNA ausgelegt sind.

10. Verfahren nach einem der Ansprüche 1 bis 9, oder Kit nach einem der Ansprüche 7 bis 9, wobei der eine oder mehrere Hoch-Risiko-HPV-Subtypen aus einer Gruppe ausgewählt sind, welche aus HPV-16, HPV-18, HPV-26, HPV-31, HPV-33, HPV-35, HPV-39, HPV-45, HPV-51, HPV-52, HPV-53, HPV-56, HPV-58, HPV-59, HPV-66, HPV-68, HPV-73 und HPV-82, oder einer beliebigen Kombination davon besteht.

11. Verfahren oder Kit nach Anspruch 10, wobei die Gruppe aus den HPV-Subtypen besteht: HPV-16, HPV-18, HPV-26, HPV-31, HPV-33, HPV-35, HPV-39, HPV-45, HPV-51, HPV-52, HPV-53, HPV-56, HPV-58, HPV-59, HPV-66, HPV-68, HPV-73 und HPV-82.

12. Verfahren oder Kit nach Anspruch 10, wobei die Gruppe aus den HPV-Subtypen besteht: HPV-16, HPV-18, HPV-31, HPV-33, HPV-35, HPV-39, HPV-45, HPV-51, HPV-52, HPV-56, HPV-58, HPV-59, HPV-68, HPV-73 und HPV-82.

13. Verfahren oder Kit nach Anspruch 10, wobei die Gruppe aus den HPV-Subtypen besteht: HPV-16, HPV-18, HPV-31, HPV-33, HPV-35, HPV-52 und HPV-58.

14. Verfahren oder Kit nach Anspruch 10, wobei die Gruppe aus HPV-Subtyp HPV-16 besteht.

## Revendications

1. Procédé de détermination si un cancer de la tête et du cou chez un être humain est associé à un PVH, comprenant la réalisation d'un test d'hybridation *in situ* (HIS) d'ARN sur un échantillon de cancer de la tête et du cou obtenu auprès dudit être humain, dans lequel ledit échantillon est dans une coupe tissulaire, dans lequel ledit test HIS comprend :
(i) plus d'un ensemble de sondes cibles qui sont conçues pour s'hybrider individuellement à l'ARNm E6/E7 de plus d'un sous-type de PVH à haut risque, et
(ii) un système d'amplification de signal universel,
dans lequel chaque ensemble de sondes cibles contient une pluralité de sondes cibles,
dans lequel chaque sonde cible au sein d'un ensemble de sondes cibles comprend une première séquence polynucléotidique qui est complémentaire de l'ARNm E6/E7 d'un sous-type de PVH à haut risque, et une seconde séquence polynucléotidique qui est complémentaire d'une séquence polynucléotidique du système d'amplification de signal universel, et
dans lequel ladite seconde séquence polynucléotidique est identique parmi les différents ensembles de sondes cibles, et
dans lequel la présence d'ARNm E6/E7 desdits un ou plusieurs sous-types de PVH à haut risque indique que le cancer de la tête et du cou chez ledit être humain est associé à un PVH.

2. Procédé de détermination de la progression d'un cancer de la tête et du cou chez un être humain, comprenant :
(a) la réalisation d'un test d'hybridation *in situ* (HIS) d'ARN sur un échantillon de cancer de la tête et du cou obtenu auprès dudit être humain, dans lequel ledit échantillon est dans une coupe tissulaire, dans lequel ledit test HIS comprend :
(i) plus d'un ensemble de sondes cibles qui sont conçues pour s'hybrider individuellement à l'ARNm E6/E7 de plus d'un sous-type de PVH à haut risque, et
(ii) un système d'amplification de signal universel ; et
(b) la mesure des taux d'ARNm E6/E7 desdits un ou plusieurs sous-types de PVH à haut risque détectés dans l'étape (a) ;
dans lequel chaque ensemble de sondes cibles contient une pluralité de sondes cibles,
dans lequel chaque sonde cible au sein d'un ensemble de sondes cibles comprend une première séquence polynucléotidique qui est complémentaire de l'ARNm E6/E7 d'un sous-type de PVH à haut risque, et une seconde séquence polynucléotidique qui est complémentaire d'une séquence polynucléotidique du système d'amplification de signal universel, et
dans lequel ladite seconde séquence polynucléotidique est identique parmi les différents ensembles de sondes cibles, et
dans lequel les taux d'ARNm E6/E7 desdits un ou plusieurs sous-types de PVH à haut risque déterminent la progression dudit cancer de la tête et du cou.

3. Procédé de détermination si une lésion cervicale chez un être humain est une lésion bénigne ou une lésion de néoplasie cervicale intraépithéliale (NCIE), comprenant la réalisation d'un test d'hybridation *in situ* (HIS) d'ARN sur un échantillon de tissu cervical obtenu auprès dudit être humain, dans lequel ledit échantillon est dans une coupe tissulaire, dans lequel ledit test HIS comprend :
(i) plus d'un ensemble de sondes cibles qui sont conçues pour s'hybrider individuellement à l'ARNm E6/E7 de plus d'un sous-type de PVH à haut risque,
(ii) un système d'amplification de signal universel ;
dans lequel chaque ensemble de sondes cibles contient une pluralité de sondes cibles,
dans lequel chaque sonde cible au sein d'un ensemble de sondes cibles comprend une première séquence polynucléotidique qui est complémentaire de l'ARNm E6/E7 d'un sous-type de PVH à haut risque, et une seconde séquence polynucléotidique qui est complémentaire d'une séquence polynucléotidique du système d'amplification de signal universel, et
dans lequel ladite seconde séquence polynucléotidique est identique parmi les différents ensembles de sondes cibles, et
dans lequel l'absence d'ARNm E6/E7 de tous lesdits sous-types de PVH à haut risque indique une lésion bénigne, et la présence d'ARNm E6/E7 desdits un ou plusieurs sous-types de PVH à haut risque indique une lésion de NCIE.

4. Procédé de détermination de la progression d'une néoplasie cervicale intraépithéliale (NCIE) chez un être humain, comprenant :
(a) la réalisation d'un test d'hybridation *in situ* (HIS) d'ARN sur un échantillon de tissu cervical obtenu auprès dudit être humain, dans lequel ledit échantillon est dans une coupe tissulaire, dans lequel ledit test HIS comprend :
(i) plus d'un ensemble de sondes cibles qui sont conçues pour s'hybrider individuellement à l'ARNm E6/E7 de plus d'un sous-type de PVH à haut risque, et
(ii) un système d'amplification de signal universel ; et
(b) l'analyse du profil spatial et la mesure des taux de l'ARNm E6/E7 desdits un ou plusieurs sous-types de PVH à haut risque détectés dans l'étape (a) ;
dans lequel chaque ensemble de sondes cibles contient une pluralité de sondes cibles,
dans lequel chaque sonde cible au sein d'un ensemble de sondes cibles comprend une première séquence polynucléotidique qui est complémentaire de l'ARNm E6/E7 d'un sous-type de PVH à haut risque, et une seconde séquence polynucléotidique qui est complémentaire d'une séquence polynucléotidique du système d'amplification de signal universel, et
dans lequel ladite seconde séquence polynucléotidique est identique parmi les différents ensembles de sondes cibles, et
dans lequel le profil spatial et les taux d'ARNm E6/E7 desdits un ou plusieurs sous-types de PVH à haut risque déterminent la progression de la NCIE.

5. Procédé de détermination du risque de développer un cancer cervical chez un être humain diagnostiqué avec une néoplasie cervicale intraépithéliale (NCIE), comprenant la réalisation d'un test d'hybridation *in situ* (HIS) d'ARN sur un échantillon de tissu cervical obtenu auprès dudit être humain, dans lequel ledit échantillon est dans une coupe tissulaire, dans lequel ledit test HIS comprend :
(i) plus d'un ensemble de sondes cibles qui sont conçues pour s'hybrider individuellement à l'ARNm E6/E7 de plus d'un sous-type de PVH à haut risque, et
(ii) un système d'amplification de signal universel ;
dans lequel chaque ensemble de sondes cibles contient une pluralité de sondes cibles,
dans lequel chaque sonde cible au sein d'un ensemble de sondes cibles comprend une première séquence polynucléotidique qui est complémentaire de l'ARNm E6/E7 d'un sous-type de PVH à haut risque, et une seconde séquence polynucléotidique qui est complémentaire d'une séquence polynucléotidique du système d'amplification de signal universel, et
dans lequel ladite seconde séquence polynucléotidique est identique parmi les différents ensembles de sondes cibles,
dans lequel lesdits un ou plusieurs sous-types de PVH à haut risque sont organisés en trois groupes de sous-types de PVH chacun avec un taux différent de risque de transformation d'une NCIE en un cancer cervical, et
dans lequel la présence d'ARNm E6/E7 des sous-types de PVH du groupe (1) indique le risque le plus élevé de développer un cancer cervical, la présence d'ARNm E6/E7 des sous-types de PVH du groupe (2) indique un risque moindre de développer un cancer cervical, et la présence d'ARNm E6/E7 des sous-types de PVH du groupe (3) indique le risque le plus bas de développer un cancer cervical ; et dans lequel lesdits sous-types de PVH du groupe (1) comportent le PVH-16 ; lesdits sous-types de PVH du groupe (2) comportent le PVH-18, le PVH-31 ; et le PVH-33 ; et lesdits sous-types de PVH du groupe (3) comportent le PVH-26, le PVH-35, le PVH-39, le PVH-45, le PVH-51, le PVH-52, le PVH-53, le PVH-56, le PVH-58, le PVH-59, le PVH-66, le PVH-68, le PVH-73, et le PVH-82.

6. Procédé selon l'une quelconque des revendications 1 à 5, dans lequel ladite coupe tissulaire est une coupe tissulaire fixée au formol, englobée dans de la paraffine.

7. Kit comprenant, dans un moyen formant récipient approprié, des agents pour réaliser un test d'hybridation *in situ* (HIS) d'ARN comportant
(i) plus d'un ensemble de sondes cibles qui sont conçues pour s'hybrider individuellement à l'ARNm E6/E7 de plus d'un sous-type de PVH à haut risque, et
(ii) un système d'amplification de signal universel,
dans lequel chaque ensemble de sondes cibles contient une pluralité de sondes cibles,
dans lequel chaque sonde cible au sein d'un ensemble de sondes cibles comprend une première séquence polynucléotidique qui est complémentaire de l'ARNm E6/E7 d'un sous-type de PVH à haut risque, et une seconde séquence polynucléotidique qui est complémentaire d'une séquence polynucléotidique du système d'amplification de signal universel, et
dans lequel ladite seconde séquence polynucléotidique est identique parmi les différents ensembles de sondes cibles, et
dans lequel le kit est destiné à déterminer si un cancer de la tête et du cou chez un être humain est associé à un PVH, ou si une lésion cervicale chez un être humain est une lésion bénigne ou une lésion de néoplasie cervicale intraépithéliale (NCIE).

8. Procédé selon l'une quelconque des revendications 1 à 6, ou kit selon la revendication 7, dans lesquels ledit système d'amplification de signal universel contient un marqueur soit de peroxydase de raifort (HRP) soit de phosphatase alcaline (PA), qui peut être détecté par la formation d'un précipité à la suite de l'incubation respectivement avec des substrats DAB ou Fast Red.

9. Procédé selon l'une quelconque des revendications 1 à 6, ou kit selon la revendication 7, dans lesquels ledit système d'amplification de signal universel contient des amplificateurs et des pré-ampfificateurs qui sont conçus pour aider à amplifier les signaux provenant de l'ARNm E6/E7.

10. Procédé selon l'une quelconque des revendications 1 à 9, ou kit selon l'une quelconque des revendications 7 à 9, dans lesquels lesdits un ou plusieurs sous-types de PVH à haut risque sont choisis dans un groupe constitué des PVH-16, PVH-18, PVH-26, PVH-31, PVH-33, PVH-35, PVH-39, PVH-45, PVH-51, PVH-52, PVH-53, PVH-56, PVH-58, PVH-59, PVH-66, PVH-68, PVH-73, et PVH-82, ou l'une quelconque de leurs combinaisons.

11. Procédé ou kit selon la revendication 10, dans lequel le groupe est constitué des sous-types de PVH de : PVH-16, PVH-18, PVH-26, PVH-31, PVH-33, PVH-35, PVH-39, PVH-45, PVH-51, PVH-52, PVH-53, PVH-56, PVH-58, PVH-59, PVH-66, PVH-68, PVH-73, et PVH-82.

12. Procédé ou kit selon la revendication 10, dans lequel ledit groupe est constitué des sous-types de PVH de : PVH-16, PVH-18, PVH-31, PVH-33, PVH-35, PVH-39, PVH-45, PVH-51, PVH-52, PVH-56, PVH-58, PVH-59, PVH-68, PVH-73, et PVH-82.

13. Procédé ou kit selon la revendication 10, dans lequel ledit groupe est constitué des sous-types de PVH de : PVH-16, PVH-18, PVH-31, PVH-33, PVH-35, PVH-52, et PVH-58.

14. Procédé ou kit selon la revendication 10, dans lequel ledit groupe est constitué du sous-type de PVH PVH-16.
